(19) Europäisches Patentamt
European Patent Office
Office européen des brevets

(11) EP 1 366 763 A1

(12) **EUROPEAN PATENT APPLICATION**

(43) Date of publication:
**03.12.2003 Bulletin 2003/49**

(21) Application number: **03405347.0**

(22) Date of filing: **20.05.2003**

(51) Int Cl.[7]: **A61K 31/09**, A61K 31/11, A61K 31/12, A61K 31/19, A61P 29/00, A61P 37/08, A61P 17/16, C07C 43/295, C07C 49/84, C07C 47/575

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **28.05.2002 EP 02405426**

(71) Applicant: **Ciba Specialty Chemicals Holding Inc.**
**4057 Basel (CH)**

(72) Inventors:
- **Baschong, Werner**
**4056 Basel (CH)**
- **Luther, Helmut**
**79639 Grenzach-Wyhlen (DE)**
- **Ochs, Dietmar**
**79650 Schopfheim (DE)**
- **Mongiat, Sébastien**
**68510 Sierentz (FR)**

(54) **Hydroxy diphenyl ether compounds as anti-inflammatory agents**

(57) Disclosed is the use of hydroxydiphenylether compounds of formula

(1) 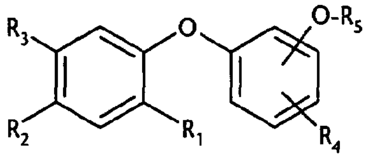 ,

, wherein

$R_1$, $R_2$ and $R_3$, independently from each other are hydrogen; hydroxy; $C_1$-$C_{20}$alkyl; hydroxy-substituted $C_1$-$C_{20}$alkyl; $C_5$-$C_7$cycloalkyl; $C_1$-$C_{20}$alkoxy; $C_1$-$C_6$alkylcarbonyl; phenyl; or phenyl-$C_1$-$C_3$alkyl;

$R_4$ is hydrogen, $C_1$-$C_{20}$alkyl; hydroxy-substituted $C_1$-$C_{20}$alkyl; $C_5$-$C_7$cycloalkyl; hydroxy; formyl; acetonyl; allyl; carboxy; carboxy-$C_1$-$C_3$alkyl; carboxyallyl; $C_2$-$C_{20}$alkenyl; $C_1$-$C_6$-alkyl-carbonyl; $C_1$-$C_3$alkylcarbonyl-$C_1$-$C_3$alkyl; phenyl; or phenyl-$C_1$-$C_3$alkyl; and

$R_5$ is hydrogen; $C_1$-$C_{20}$alkoxy; or $C_1$-$C_6$alkylcarbonyl;

as pharmaceutical active agents and to pharmaceutical compositions containing them.

EP 1 366 763 A1

## Description

The present invention relates to the use of hydroxydiphenylether compounds as pharmaceutical active agents and to pharmaceutical compositions containing them. The invention further relates to these compounds for the preparation of medicaments for the treatment of inflammatory and allergic conditions.
It is standard practice to use glucocorticoids for the topical treatment of inflammatory and allergic conditions. It is common knowledge that these compounds can have unwanted side-effects.
Owing to their insufficient ability to penetrate the skin, nonsteroidal antiinflammatory medicaments containing therapeutic agents such as ketoprofen, BW755c, piroxicam, diclofenac or indomethazin cannot effectively be applied topically, but only systemically (q.v. inter alia G.B. Kasting et al., Pharmacol. Skin., Vol.1, pp. 138-153, Karger, Basel 1987).
It is the object of this invention to provide pharmaceutical compositions having pharmacologically useful properties, in particular antioxidant, anti-inflammatory and antiallergic properties, especially when administered locally.
Surprisingly, it has been found that hydroxydiphenylether compounds of formula

(1) 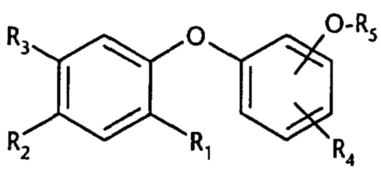 ,

wherein

| | |
|---|---|
| $R_1$, $R_2$ and $R_3$, | independently from each other are hydrogen; hydroxy; $C_1$-$C_{20}$alkyl; hydroxy-substituted $C_1$-$C_{20}$ alkyl; $C_5$-$C_7$cycloalkyl; $C_1$ -$C_{20}$alkoxy; $C_1$-$C_6$alkylcarbonyl; phenyl; or phenyl-$C_1$-$C_3$alkyl; |
| $R_4$ | is hydrogen, $C_1$-$C_{20}$alkyl; hydroxy-substituted $C_1$-$C_{20}$alkyl; $C_5$-$C_7$cycloalkyl; hydroxy; formyl; acetonyl; allyl; carboxy; carboxy-$C_1$-$C_3$alkyl; carboxyallyl; $C_2$-$C_{20}$alkenyl; $C_1$-$C_6$alkyl-carbonyl; $C_1$-$C_3$alkylcarbonyl-$C_1$-$C_3$alkyl; phenyl; or phenyl-$C_1$-$C_3$alkyl; and |
| $R_5$ | is hydrogen; $C_1$-$C_{20}$alkoxy; or $C_1$-$C_6$alkylcarbonyl; |

exhibit marked antiinflammatory action in cellular and enzymatic in vitro assays and in in vivo assays on human volunteers.
$C_1$-$C_{20}$alkyl is straight-chain or branched alkyl radicals such as methyl, ethyl, n-propyl, isopropyl, n-butyl, sec-butyl, tert-butyl, pentyl, iso-pentyl, tert-pentyl, hexyl, cyclohexyl, heptyl, octyl, isooctyl, nonyl or decyl and the like.
$C_1$-$C_{20}$ alkoxy is straight-chain or branched alkoxy radicals such as methoxy, ethoxy, n-propoxy, isopropoxy, n-butoxy, sec-butoxy, tert-butoxy, pentyloxy, iso-pentyloxy, tert-pentyloxy, heptyloxy, octyloxy, isooctyloxy, nonyloxy or decyloxy and the like.
$C_1$-$C_6$ alkyl carbonyl is straight-chain or branched carbonyl radicals such as acetyl, propionyl, butyryl, isobutyryl, valeryl, isovaleryl or pivaloyl and the like.
Hydroxy substituted $C_1$-$C_{20}$alkyl is hydroxymethyl, hydroxyethyl, hydroxypropyl, hydroxybutyl, hydroxypentyl, hydroxyhexyl, hydroxyheptyl, hydroxyoctyl, hydroxynonyl or hydroxydecyl and the like.
Those compounds of formula (1) have proved particularly interesting in which

| | |
|---|---|
| $R_1$, $R_2$ and $R_3$, | independently of each other, are hydrogen; $C_1$-$C_{20}$alkyl; hydroxy-substituted $C_1$-$C_{20}$alkyl; or $C_1$-$C_{20}$alkoxy; |
| $R_4$ | is hydrogen; $C_1$-$C_{20}$ alkyl; hydroxy-substituted $C_1$-$C_{20}$ alkyl; hydroxy; formyl; acetonyl; allyl; carboxymethyl; or carboxyallyl; and |
| $R_5$ | is hydrogen; $C_1$-$C_{20}$alkoxy; or $C_1$-$C_6$alkylcarbonyl; |

preferably those, wherein at least one of the substituents $R_1$, $R_2$, $R_3$ and $R_4$ is not hydrogen.
Compounds of very particular interest are those of formula (1) wherein

| | |
|---|---|
| $R_1$ | is $C_1$-$C_{16}$alkyl; and |
| $R_2$, $R_3$ $R_4$ and $R_5$ | are hydrogen. |

Compounds of very most particular interest are those of formula

(1a) 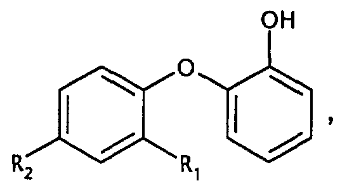

wherein

$R_1$ and $R_2$ independently of each other are hydrogen; $C_1$-$C_{20}$alkyl; or hydroxy-substituted $C_1$-$C_{20}$alkyl;

preferably those, wherein at least one of the substituents $R_1$ and $R_2$ is not hydrogen.

Furthermore, compounds of formula

(1b) 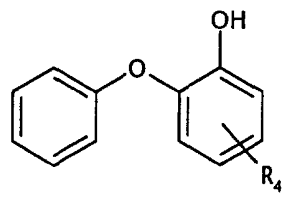

are of particular interest, wherein

$R_1$ is carboxy; carboxy-$C_1$-$C_3$alkyl; $C_1$-$C_3$alkylcarbonyl; or $C_1$-$C_3$alkylcarbonyl-$C_1$-$C_3$-alkyl.

Furthermore, compounds of formula

(1c) 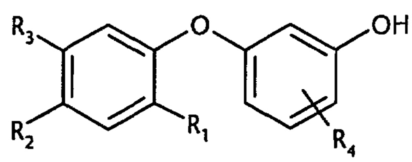

are of particular interest, wherein

$R_1$, $R_2$ and $R_3$ independently from each other are hydrogen; hydroxy; $C_1$-$C_{20}$ alkyl; or hydroxy-substituted $C_1$-$C_{20}$alkyl; and

$R_4$ is C,-$C_{20}$alkyl; hydroxysubstituted $C_1$-$C_{20}$; Phenyl; Phenyl-$C_1$-$C_3$alkyl; or $C_1$- $C_3$alkylcarbonyl;

and most preferably compounds of formula (1c), wherein
$R_1$, $R_2$ and $R_3$ are hydrogen; or $C_1$-$C_{20}$alkyl.

Most preferably are compounds of formula (1c), wherein

$R_1$, $R_2$ and $R_3$ are hydrogen; and

$R_4$ is $C_1$-$C_{20}$alkyl; phenyl-$C_1$-$C_3$alkyl; or $C_1$-$C_6$alkylcarbonyl.

Furthermore, compounds of formula

(1d) 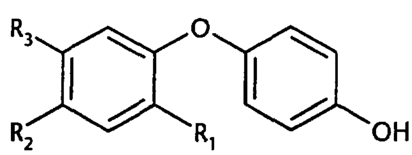

are preferably used, wherein

$R_1$, $R_2$, and $R_3$, independently from each other are hydrogen; $C_1$-$C_{20}$alkyl; hydroxy-substituted $C_1$-$C_{20}$alkyl; cyclo-$C_5$-$C_7$alkyl; phenyl-$C_1$-$C_3$alkyl;

Preferably those, wherein at least one of the substituents $R_1$, $R_2$, and R is not hydrogen.

Mostly preferred are compounds of formula (1d), wherein
$R_1$ and $R_2$, independently of each other are $C_1$-$C_{20}$alkyl; most preferably $C_1$-$C_5$alkyl; and
$R_3$ is hydrogen.

Further hydroxydiphenylehter compounds, which can be used in the present invention, are listed in the following Table 1:

| Table 1: | | | |
|---|---|---|---|
| HD-01 | 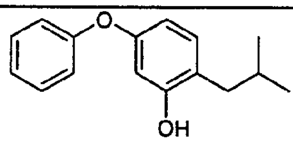 | HD-02 | 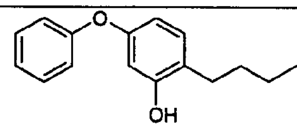 |
| HD-03 | 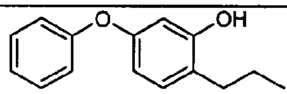 | HD-04 | 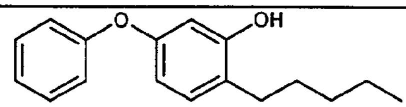 |
| HD-05 | 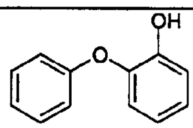 | HD-06 | 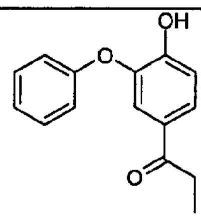 |
| HD-07 | 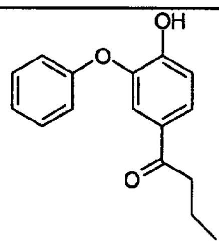 | HD-08 | 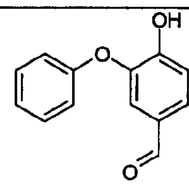 |
| HD-09 | 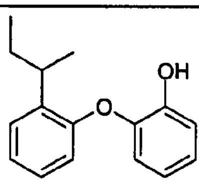 | HD-10 | 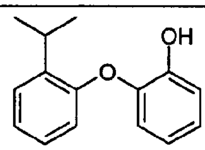 |
| HD-11 | 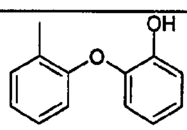 | HD-12 | 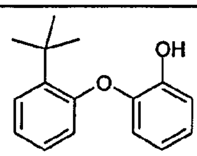 |

Table 1:

| | | | |
|---|---|---|---|
| HD-13 | 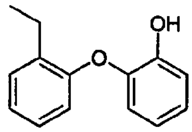 | HD-14 | 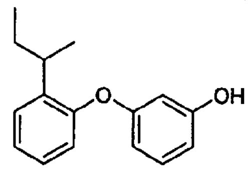 |
| HD-15 | 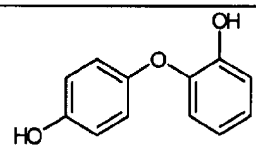 | HD-16 | 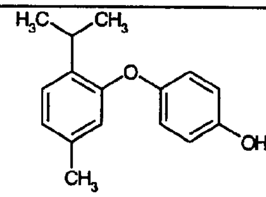 |
| HD-17 | 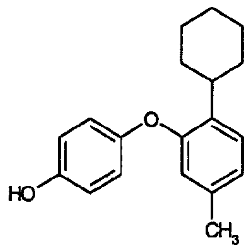 | HD-18 | 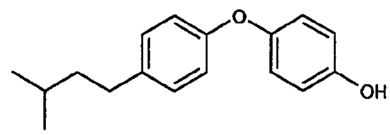 |
| HD-19 | 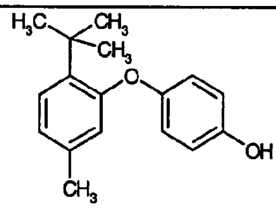 | HD-20 | 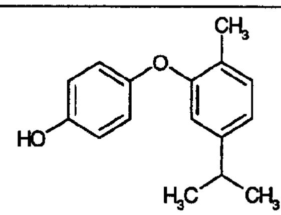 |
| HD-21 | 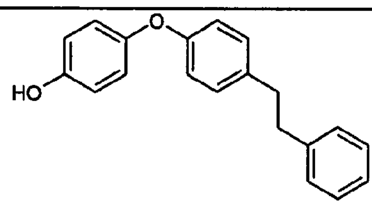 | HD-22 | 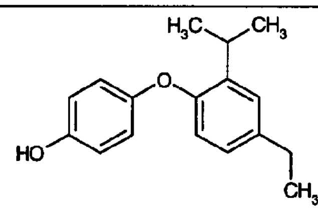 |
| HD-23 | 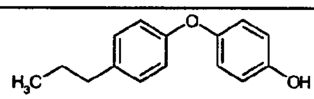 | HD-24 | 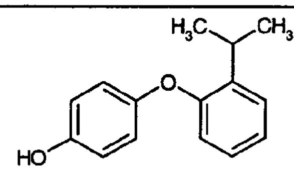 |

Table 1:

| | | | |
|---|---|---|---|
| HD-25 | 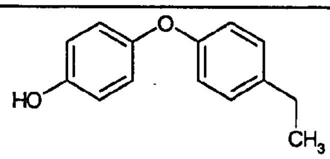 | HD-26 | 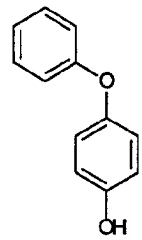 |
| HD-27 | 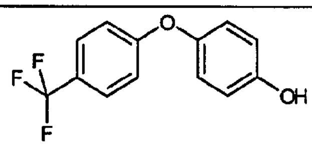 | HD-28 | 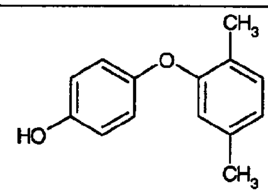 |
| HD-29 | 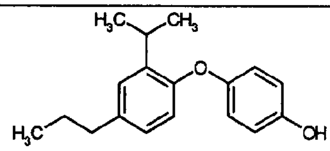 | HD-30 | 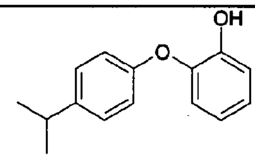 |
| HD-31 | 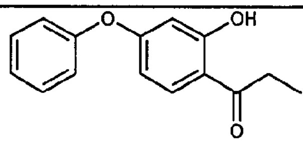 | HD-32 | 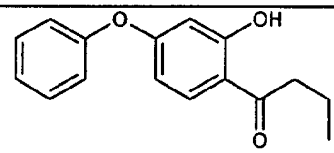 |
| HD-33 | 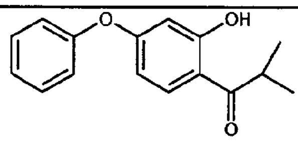 | HD-34 | 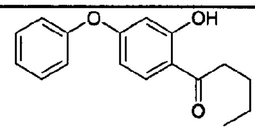 |
| HD-35 | 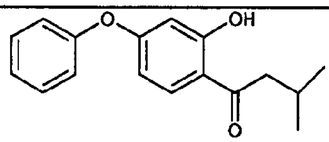 | HD-36 | 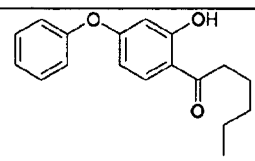 |
| HD-37 | 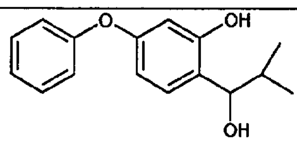 | HD-38 | 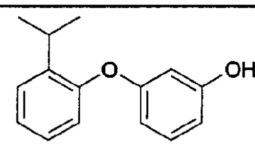 |
| HD-39 | 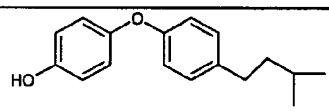 | HD-40 | 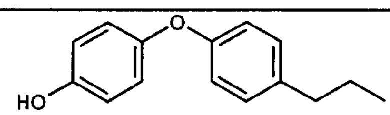 |

Table 1:

| | | | |
|---|---|---|---|
| HD-41 | 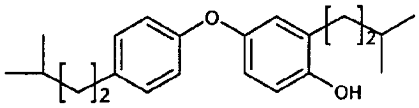 | HD-42 | 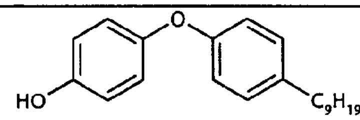 |
| HD-43 | 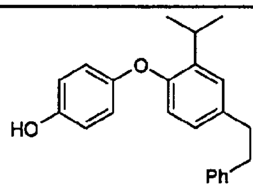 | HD-44 | 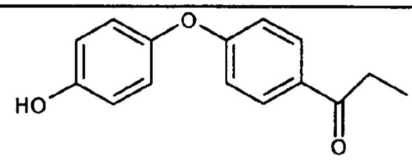 |
| HD-45 | 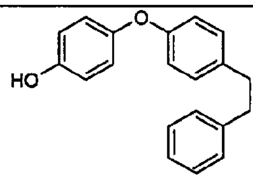 | HD-46 | 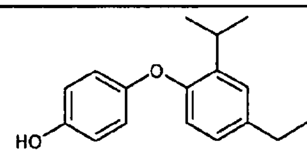 |
| HD-47 | 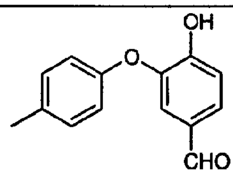 | HD-48 | 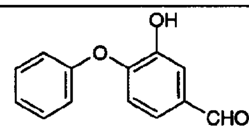 |
| HD-49 | 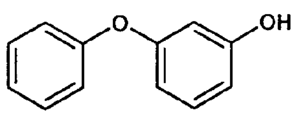 | HD-50 | 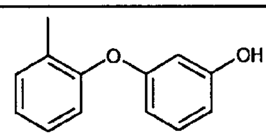 |
| HD-51 | 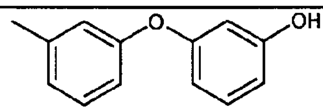 | HD-52 | 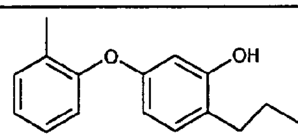 |
| HD-53 | 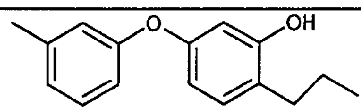 | HD-54 | 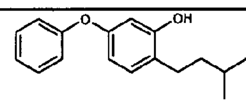 |
| HD-55 | 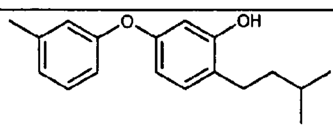 | HD-56 | 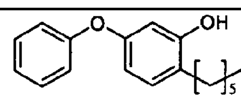 |
| HD-57 | 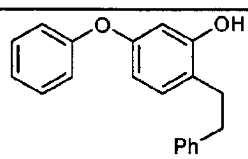 | HD-58 | 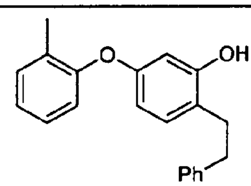 |

| Table 1: | | | |
|---|---|---|---|
| HD-59 | 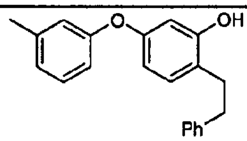 | HD-60 | 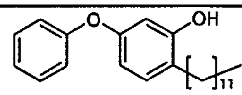 |
| HD-61 | 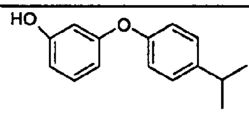 | HD-62 | 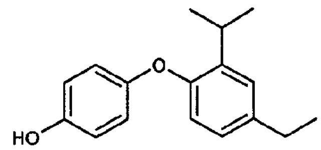 |
| HD-63 | 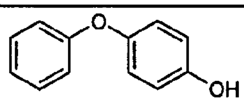 | HD-64 | 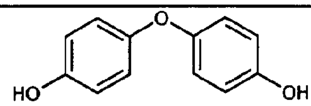 |
| HD-65 | 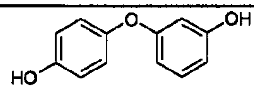 | HD-66 | 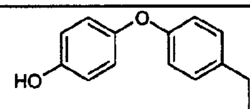 |

The compounds of formula (1) are useful for the treatment of inflammatory and allergic conditions, as well as for the treatment of conditions involving disturbances of cell proliferation.

In vitro assays show that the compounds of formula (1) inhibit the formation of different mediators that are an important factor in inflammation.

The compounds of formula (1) take effect as radical inhibitor. They represent lipoxygenase/cyclooxigenase inhibitors, i.e. they can intervene in the inflammatory cascade. It can be shown that they take effect as anti-inflammatory agent for the UV induced erythema. They show anti-inflammatory and antioxidant effect comparable to vitamin E.

A further subject of the present invention is a pharmaceutical composition comprising at least one compound of formula (1), together with a pharmaceutically acceptable carrier or adjuvant.

The pharmaceutical compositions are preferably liquid, semisolid or solid preparations.

Examples of liquid pharmaceutical compositions are injectable solutions, infusion solutions, drops, sprays, aerosols, emulsions, lotions, suspensions, drinking solutions, gargles and inhalants.

Examples of semisolid pharmaceutical compositions are ointments, creams (O/W emulsions), rich creams (W/O emulsions), gels, lotions, foams, pastes, suspensions, ovula, plasters, including transdermal systems.

Examples of solid pharmaceutical compositions are tablets, coated tablets, capsules, granules, effervescent granules, effervescent tablets, lozenges, sucking and chewing tablets, suppositories, implants, lyophilisates, adsorbates or powders.

Galenic pharmaceutical compositions comprising the compounds of formula (1) will be understood as meaning in particular emulsions, ointments, gels, sprays and powders.

Compounds of formula (1) may also be contained in liposomes or used in pharmacological compositions with conventional carriers and penetration enhancers, for example urea, dextrane, propylene glycol, oleic acid and the like.

The pharmaceutical composition will usually contain the compounds of formula (1) in amounts of 0.001 to 10 % by weight, preferably of 0.1 to 5% by weight, of the total mixture. For the treatment of the conditions listed hereinabove, the pharmaceutical composition of this invention may contain, in addition to the compounds of formula (1), further pharmaceutical agents having antiphlogistic activity, typically including antiinflammatory agents, antipsoriatic agents, cell proliferation regulators, and antiallergic, gastroprotective and antiasthmatic agents.

The following Examples will serve to illustrate the invention without implying any restriction to what is described therein. Unless otherwise indicated, percentages are by weight.

Example 1: Evaluation of the Efficacy of hydroxvdiphenylether compounds against Photooxidative Stress in the Skin induced by UVA Irradiation

| | Trade Name | INCI-Name | Placebo | Formulation 1A | Formulation 1A |
|---|---|---|---|---|---|
| Part A | Cutina GMS | Glyceryl stearate | 4.50 | 4.50 | |
| | Nexbase 2006 FG | Hydrogenated Polydecene | 5.00 | 5.00 | 5.00 |
| Part B | Water Deionized | Aqua | qs 100 | qs 100 | qs 100 |
| Part C | Compound of formula (101) | | | 0.10 | 0.5 |
| | Propylene Glycol | Propylene Glycol | 5.00 | 5.00 | 5.00 |
| Part D | Caramel Color | | 0.02 | 0.02 | 0.02 |
| | NaOH 10% | Sodium Hydroxide | qs | qs | Qs |
| | Phenonip | Phenoxyethanol(and) Methylparaben(and) Ethylparaben(and) Butylparaben(and) Propylparaben (and) Isobutylparaben | 0.60 0.60 | 0.60 0.60 | 0.60 0.60 |
| | Salcare SC96 | Polyquaternium-37, Propylene Glycol Dicaprylate, PPG-1 Trideceth-6 | 0.80 | 0.80 | 0.80 |

Formula (101): 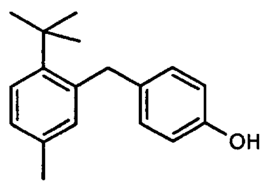

Preparation method:

The compound of formula (101) is dispersed in propylene glycol at 75°C.
Phase B is heated at 75°C. Before the emulsification, phase C is poured into phase A. Phase A is then poured into phase B under moderate stirring.
The mixture is homogenised with an Ultra Turrax for 10s.
Then the mixture is cooled down to 60°C with a very moderate stirring.
Salcare SC96 is incorporated into the emulsion and Phenonip added under stirring. Stirring is continued till 40°C, the emulsion colored and pH-adjusted afterwards.

| | |
|---|---|
| Persons tested: | 10 individuals (4 female, 6 male); age range: 28 to 50 years |
| Body region tested: | back |
| Application phase: | twice a day |
| Test period | 7 days |
| Evaluation method: | -Determination of squalene<br>-Determination of squalenehydroperoxide |
| Time of Evaluation: | after UVA radiation |
| Descriptive statistics: | average, median, minimum, maximum, variance, standard error, standard deviation; Multiple |

range test.

Test method

On the back of each subject symmetrically opposed areas are defined. The different formulations are applied twice a day at a dose of about 2 mg/$cm^2$ for one week (application with a syringe for fine dosage: Omnifix® - F 1 ml; manufacturer: Braun Melsungen AG, Germany).

Two areas remain untreated.

The unique application of a solution of tocopherol in ethanol (0,2%) before irradiation serves as control.

Use of other cosmetic products is restricted on the test areas throughout the whole study. The areas (exception: one of the two untreated areas was not irradiated and can be attributed to environmental UVA radiation during the test) of the subject's back were then irradiated with UVA light (10 joule/ $cm^2$). The lipids present on the test areas are harvested via a solvent extraction (4 ml ethanol for 2 minutes). The samples are first filtered through hydrophobic polypropylene filters to decant squames and other insoluble material, then dried under nitrogen at room temperature and taken up in 1 ml ethanol. Squalene (SQ) and squalenehydroperoxide (SQOOH) are then analysed by High Performance Liquid Chromatography (HPLC).

The results are expressed as the rate of inhibition relative to the untreated area:

$$\% \text{ inhibition} = 100 \times [\text{SQOOH(untreated)} - \text{SQOOH(active)}] / \text{SQOOH(untreated)}$$

(SQOOH in pmoles hydrogen peroxide per pg squalene)

HPLC analysis for SQ

| | |
|---|---|
| column: | LiChrospher® 100 RP-18 (5µm, 125 x 4 mm) Merck-Germany mobile phase: acetonitrile/isopropanol (1 /1; VN) |
| detection: | UV 210 nm |
| flow rate: | 1 ml/min |
| injection volume: | 20µl |
| equipment: | Beckman System Gold (USA) with Programmable Solvent Module 126 and Programmable Detector Module 166 |

HPLC analysis for SQOOH

| | |
|---|---|
| column: | LiChrospher® 100 RP-Select B (5µm, 125 x 4 mm) Merck-Germany |
| mobile phase: | methanol |
| detection: | Chemiluminescence (post column detection) |
| flow rate: | 1 ml/min |
| injection volume: | 20µl |
| reaction solution: | Luminol (1 µg/ml) and Cytochrom C (10 µg/ml solved in 50mM Borate-buffer, pH 10) |
| equipment: | Beckman System Gold (USA) with Programmable Solvent Module 126 and fluorometer RF-551 (Shimadzu, Japan) |

The fluorometer is used as a photon detector with the excitation source turned off.

This assay measures the hydroperoxy groups themselves and not indirect indices of lipid peroxidation such as conjugated dienes or breakdown products of lipid hydroperoxides. Chemiluminescence also detects ubiquinols. To confirm that any chemiluminescence observed in this assay was due to a hydroperoxide, not a hydroquinone, some samples were reduced with triphenyl phosphine and rerun. Since the chemiluminescence response of hydroperoxides, but not of hydroquinones, is eliminated by triphenyl phosphine, the disappearance of chemiluminescence peaks in the treated samples indicated that chemiluminescence observed in this assay was due to hydroperoxides and not hydroquinones.

Biometry

Measuring data are centrally computerised after validity check and quality assurance. Evaluation is done using the software Statgraphics for Windows, Version 5.0 - Manugistics, USA. Data are analysed by multiple range test (LSD: Least Significant Differences). The 0.05 level is selected as the point of minimal acceptance statistical significance.

Results

The skincare formulations 1 A and 1 B prevented squalene peroxidation.
In the untreated, non-irradiated area the formation of ethanol extractable squalenehydroperoxide is not detected.
The greatest efficacy has formulation 1 B.
The average inhibition in % of peroxidation was in the order of:

| Formulation | Inhibition |
|---|---|
| Placebo | 7 ± 1 |
| Formulation 1A | 13 ± 5 |
| Formulation 1B | 25 ±7 |
| Control | 24 ± 4 |

Statistically significant differences between the two formulations, the control and the untreated area ccan be detected:

| Formulation | Placebo | 1A | 1B | Control | Untreated |
|---|---|---|---|---|---|
| Placebo | - | s. | s. | s. | s. |
| 1A | s. | - | s. | | s. |
| 1B | s. | s. | - | | s. |
| Control | s. | s. | n.s. | - | s. |
| Untreated | s. | s. | s. | | - |

The Placebo shows only a minor efficacy against UVA induced squalene peroxidation. In the untreated area squalene peroxidation increased tremendously.

Conclusion:

Pretreatment of the epidermal surface before exposure to UVA irradiation with the formulations according to the present invention prevents an increase in sebum peroxidation. These studies provide compelling evidence for the free radical hypothesis of UVA light induced cutaneous pathology lipid peroxidation increased on irradiation and the topical application with an antioxidant system prevented this damage.

B. Preparation of pharmaceutical compositions

Example 2:

An ointment containing 2 % of the compound of formula (101) can be prepared as follows:

| Composition: | |
|---|---|
| active ingredient | 2 % |
| vaseline | 45.0 % |
| paraffin oil | 19.6 % |
| cetyl alcohol | 5.00 % |
| beeswax | 5.00 % |

(continued)

| Composition: | |
|---|---|
| sorbitan sesquioleate | 5.00 % |
| p-hydroxybenzoate | 0.20 % |
| demineralised water to make up | 100.00 % |

The fatty substances and emulsifiers are fused together and the active ingredient is dissolved therein. The preservative is dissolved in water and the solution is emulsified at elevated temperature into the melt and the emulsion is then stirred until cold.

Example 3:

A cream containing 1 % of the compound of formula (101) can be prepared as follows:

| Composition: | |
|---|---|
| active ingredient | 1 % |
| isopropyl palmitate | 8.0 % |
| cetyl palmitate | 1.5 % |
| silicone oil 100 | 0.5 % |
| sorbitan monostearate | 3.0 % |
| polysorbate 60 | 3.5 % |
| 1,2-propylene glycol PH | 20.0 % |
| acrylic acid polymer | 0.5 % |
| triethanolamine | 0.7 % |
| demineralised water to make up | 100.00 % |

The acrylic acid polymer is suspended in a mixture of demineralised water and 1,2-propylene glycol. With stirring, triethanolamine is added to form a mucilage. A mixture of isopropyl palmitate, cetyl palmitate, silicone oil, sorbitan monostearate and polysorbate is heated to ca. 75°C and the active igredient is dissolved therein. This fatty phase is stirred into the mucilage, which is also heated to c.75°C, and the batch is stirred until cold.

Example 4:

A cream containing 0.5 % of the compound of formula (101) can be prepared as follows:

| Composition: | |
|---|---|
| active ingredient | 0.5 % |
| cetyl palmitate PH | 2.00 % |
| cetyl alcohol PH | 2.00 % |
| triglyceride mixture of saturated medium fatty acids | 5.00 % |
| stearic acid | 3.00 % |
| glycerol stearate PH | 4.00 % |
| Cetomacrogol 1000 | 1.00 % |
| microcrystalline cellulose | 0.50 % |
| 1,2-propylene glycol, dist. | 20.00 vf |
| demineralised water to make up | 100.00%. |

Cetyl alcohol, cetyl palmitate, the triglyceride mixture, stearic acid and glycerol stearate are fused together and the active ingredient is dissolved therein. The microcrystal line cellulose is dispersed in some of the water. Cetomacro gol is dissolved in the remainder of the water and the propylene glycol and the mucilage are mixed therewith. The fatty phase is then stirred into the aqueous phase and stirred until cold.

The formulation is suitable for use as a moisturising skin-protective cream.

Example 5:

A transparent hydrogel containing 0.2 % of the compound of formula (101) is prepared as follows:

| Composition: | |
|---|---|
| active ingredient | 0.2 % |
| propylene glycol | 10.0-20.0 % |
| isopropanol | 20.0 % |
| hydroxypropylmethyl cellulose | 2.0 % |
| water to make up | 100.00 % |

The hydroxypropylmethyl cellulose is swollen in water. The active ingredient is dissolved in a mixture of isopropanol and propylene glycol. Then the active ingredient solution is mixed with a swollen cellulose derivative and, if desired, perfume is added (0.1%).

The formulation is suitable for use as a moisturising skin gel.

Example 6:

A transparent hydrogel containing 0.02 % of the compound of formula (101) is prepared as follows:

| Composition: | |
|---|---|
| active ingredient | 0.02 % |
| propylene glycol | 20.0 % |
| isopropanol | 20.0 % |
| acrylic acid polymer | 2.0 % |
| triethanolamine | 3.0 % |
| water | to make up 100.00 % |

Acrylic acid polymer and water are dispersed and the dispersion is neutralised with triethanolamine. The active ingredient is dissolved in a mixture of isopropanol and propylene glycol. The active ingredient solution is then mixed with a gel and, if desired, perfume oil (0.1%) can be added.

Example 7:

A foam spray containing 1 % of the compound of formula (101) can be prepared as follows:

| Composition: | |
|---|---|
| active ingredient | 1 % |
| cetyl alcohol PH | 1.70 % |
| viscous paraffin oil | 1.00 % |
| isopropyl myristate | 2.00 % |
| Cetomacrogol 1000 | 2.40 % |
| sorbitan monostearate | 1.50 % |
| 1,2-propylene glycol PH | 5.00 % |
| methyl parabene | 0.18 % |
| propyl parabene | 0.02 % |
| Chemoderm 314 | 0.10 % |
| demineralised water | to make up 100.00 % |

Cetyl alcohol, paraffin oil, isopropyl myristate, Cetomacrogol and sorbitan stearate are fused together and the active ingredient is dissolved therein. Methyl and propyl parabene are dissolved in propylene glycol and the solution is added to the hot water. The melt and the solution are afterwards mixed. After cooling, the Chemoderm is added and, if desired, perfume oil (0.1 %) the formulation is bulked with water to the final weight.

Filling:

An aluminium dispenser is filled with 20 ml of the mixture. The dispenser is fitted with a nozzle and propellant gas is introduced under pressure.

Example 8:

An eye ointment containing 0.01 % of the compound of formula (101) can be prepared as follows:

| Composition: | |
|---|---|
| active ingredient | 0.01 % |
| viscous paraffin oil | 10% |
| wool grease anhyd. | 10% |
| white vaseline | 79 % |
| demineralised water to make up | 100 % |

The constituents are fused together and filtered under sterile conditions.

Example 9:

Capsules suitable for insufflation and containing 0.025% of the compound of formula (101) can be prepared as follows:

| Composition: (for 1000 capsules): | |
|---|---|
| active ingredient | 25.00 g |
| milled lactose | 25.00 g |

The active ingredient and the microfine lactose are thoroughly blended. The powder is sieved and filled in 0.05 g portions into gelatine capsules.

**Claims**

**1.**

(1) 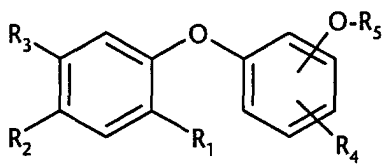 ,

wherein

$R_1$, $R_2$ and $R_3$, independently from each other are hydrogen; hydroxy; $C_1$-$C_{20}$alkyl; hydroxy-substituted $C_1$-$C_{20}$ alkyl; $C_5$-$C_7$cycloalkyl; $C_1$-$C_{20}$alkoxy; $C_1$-$C_6$alkylcarbonyl; phenyl; or phenyl-$C_1$-$C_3$alkyl;

$R_4$ is hydrogen, $C_1$-$C_{20}$alkyl; hydroxy-substituted $C_1$-$C_{20}$alkyl; $C_5$-$C_7$cycloalkyl; hydroxy; formyl; acetonyl; allyl; carboxy; carboxy-$C_1$-$C_3$alkyl; carboxyallyl; $C_2$-$C_{20}$alkenyl; $C_1$-$C_6$alkyl-carbonyl; $C_1$-$C_3$alkylcarbonyl-$C_1$-$C_3$alkyl; phenyl; or phenyl-$C_1$-$C_3$alkyl; and

$R_5$ is hydrogen; $C_1$-$C_{20}$alkoxy; or $C_1$-$C_6$alkylcarbonyl;

for use as a therapeutic agent.

**2.** A compound according to claim 1, wherein

$R_1$, $R_2$ and $R_3$, independently of each other, are hydrogen; $C_1$-$C_{20}$alkyl; hydroxy-substituted $C_1$-$C_{20}$alkyl; or $C_1$-$C_{20}$alkoxy;

$R_4$ is hydrogen; $C_1$-$C_{20}$ alkyl; hydroxy-substituted $C_1$-$C_{20}$ alkyl; hydroxy; formyl; acetonyl; allyl; car-

| | |
|---|---|
| | boxymethyl; or carboxyallyl; and |
| $R_5$ | is hydrogen; $C_1$ -$C_{20}$alkoxy; or $C_1$-$C_6$alkylcarbonyl. |

**3.** A compound according to claim 1 or 2, wherein

| | |
|---|---|
| $R_1$ | is $C_1$-$C_{16}$alkyl; and |
| $R_2$, $R_3$ $R_4$ and $R_5$ | are hydrogen. |

**4.** A compound according to claim 1, which corresponds to formula

(1a) 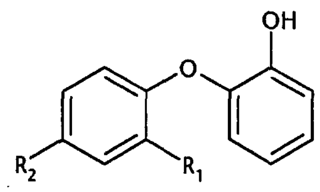 ,

wherein

$R_1$ and $R_2$ independently of each other are hydrogen; $C_1$-$C_{20}$alkyl; or hydroxy-substituted $C_1$-$C_{20}$alky.

**5.** A compound according to claim 1, which corresponds to formula

(1b) 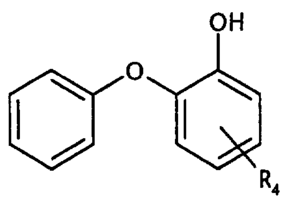

are of particular interest, wherein

R, is carboxy; carboxy-$C_1$-$C_3$alkyl; $C_1$-$C_3$alkylcarbonyl; or $C_1$-$C_3$alkylcarbonyl-$C_1$-$C_3$alkyl.

**6.** A compound according to claim 1, which corresponds to formula

(1c) 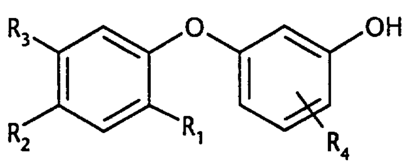 ,

wherein

| | |
|---|---|
| $R_1$, $R_2$ and $R_3$ | independently from each other are hydrogen; hydroxy; $C_1$-$C_{20}$alkyl; or hydroxy-substituted $C_1$-$C_{20}$alkyl; and |
| $R_4$ | is $C_1$-$C_{20}$alkyl; hydroxysubstituted $C_1$-$C_{20}$; Phenyl; Phenyl-$C_1$-$C_3$alkyl; or $C_1$-$C_3$alkylcarbonyl; |

**7.** A compound according to claim 6, wherein $R_1$, $R_2$ and $R_3$ are hydrogen; or $C_1$-$C_{20}$alkyl.

**8.** A compound according to claim 6 or 7, wherein

| | |
|---|---|
| $R_1$, $R_2$ and $R_3$ | are hydrogen; and |
| $R_4$ | is $C_1$ -$C_{20}$alkyl; phenyl-$C_1$-$C_3$alkyl; or $C_1$-$C_6$alkylcarbonyl. |

**9.** A compound according to claim 1, which corresponds to formula

(1d) 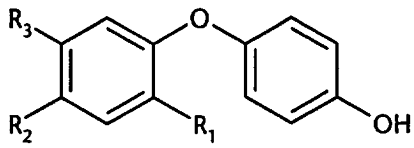 ,

wherein

$R_1$,$R_2$, and $R_3$, independently from each other are hydrogen; $C_1$-$C_{20}$alkyl; hydroxy-substituted $C_1$-$C_{20}$alkyl; cyclo-$C_5$-$C_7$alkyl; phenyl-$C_1$-$C_3$alkyl.

**10.** A compound according to claim 9, wherein

$R_1$ and $R_2$, independently of each other are $C_1$-$C_{20}$alkyl; and
$R_3$ is hydrogen.

**11.** A pharmaceutical composition comprising at least one compound as claimed in any one of claims 1 to 10, together with a pharmaceutically acceptable carrier or adjuvant.

**12.** A pharmaceutical composition according to claim 11, wherein the compound of formula (1) is present in amounts of 0.001 to 10% by weight, of the total mixture.

**13.** A pharmaceutical composition according to claim 11 or 12, which additionally comprises at least one substance having antiphlogistic action.

**14.** Use of a pharmaceutical composition according to one of claims 11,12 or 13 for the local treatment of inflammatory and allergic conditions.

**15.** Use of a compound according to any one of claims 1 to 10 for the preparation of a medicament for the treatment of radical-induced skin damage and inflammatory and allergic conditions, as well as for the treatment of conditions involving disturbances of cell proliferation.

European Patent Office

**PARTIAL EUROPEAN SEARCH REPORT**

which under Rule 45 of the European Patent Convention shall be considered, for the purposes of subsequent proceedings, as the European search report

Application Number EP 03 40 5347

**DOCUMENTS CONSIDERED TO BE RELEVANT**

| Category | Citation of document with indication, where appropriate, of relevant passages | Relevant to claim | CLASSIFICATION OF THE APPLICATION (Int.Cl.7) |
|---|---|---|---|
| X | EP 1 053 989 A (CIBA SPECIALITY CHEMICALS) 22 November 2000 (2000-11-22)<br>* page 12, line 8 - page 13, line 24; claims; table 1 *<br>--- | 1-12 | A61K31/09<br>A61K31/11<br>A61K31/12<br>A61K31/19<br>A61P29/00<br>A61P37/08<br>A61P17/16<br>C07C43/295<br>C07C49/84<br>C07C47/575 |
| X | EP 0 033 614 A (FUJISAWA PHARMACEUTICAL) 12 August 1981 (1981-08-12)<br>* page 29, example 3-(9); pages 17 and 18, tables 1 and 2; claims 1, 9, 10 *<br>--- | 1,2,11,12,15 | |
| X | US 3 600 437 A (W. S. MARSHALL) 17 August 1971 (1971-08-17)<br>* column 4, line 15 *<br>* column 1, line 54 - line 72 *<br>--- | 1,2,5,11,13,15 | |
| X | S. IGARASHI: "A novel class of inhibitors for human steroid 5-alpha-reductase: phenoxybenzoic acid derivatives. I." CHEMICAL AND PHARMACEUTICAL BULLETIN., vol. 47, no. 8, 1999, pages 1073-1080, XP002240858 TOKYO JP<br>* page 1075, compounds 2s and 2t; page 1077, table 2 *<br>--- | 1,11 | **TECHNICAL FIELDS SEARCHED (Int.Cl.7)**<br>C07C<br>A61K<br>A61P |

-/--

**INCOMPLETE SEARCH**

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 6 October 2003 | Wright, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03 82 (P04C07)

European Patent Office

**INCOMPLETE SEARCH SHEET C**

Application Number
EP 03 40 5347

Although claim 14 is directed to a method of treatment of the human/animal body (Article 52(4) EPC), the search has been carried out and based on the alleged effects of the composition.

---

Claim(s) searched completely:
1-15

Reason for the limitation of the search (non-patentable invention(s)):

Article 52 (4) EPC - Method for treatment of the human or animal body by therapy

European Patent Office

# PARTIAL EUROPEAN SEARCH REPORT

Application Number
EP 03 40 5347

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 5 185 377 A (T. SCHEWE)<br>9 February 1993 (1993-02-09)<br>* claims 1-3 *<br>--- | 1,11 | |
| X | G. ROY: "Halide and alkyl phenols block volume-sensitive chloride channels in human glial cells (U-138MG)"<br>J. MEMBRANE BIOL.,<br>vol. 162, 1998, pages 191-200, XP002240859<br>* page 196; table 4 *<br>--- | 1,2,9 | |
| P,X | WO 02 50008 A (WARNER-LAMBERT)<br>27 June 2002 (2002-06-27)<br>* page 16, line 22 - line 26 *<br>* page 19, line 24 - page 20, line 5; claims 1,2 *<br>* page 11, line 31 - page 12, line 5 *<br>----- | 1-4,11,<br>12 | TECHNICAL FIELDS SEARCHED (Int.Cl.7) |

EPO FORM 1503 03.82 (P04C10)

## ANNEX TO THE EUROPEAN SEARCH REPORT ON EUROPEAN PATENT APPLICATION NO. EP 03 40 5347

This annex lists the patent family membersrelating to the patent documents cited in the above-mentioned European search report.
The members are as contained in the European Patent Office EDP file on
The European Patent Office is in no way liable for these particulars which are merely given for the purpose of information.

06-10-2003

| Patent document cited in search report | Publication date | Patent family member(s) | Publication date |
|---|---|---|---|
| EP 1053989 A | 22-11-2000 | EP 1053989 A2 | 22-11-2000 |
| | | BR 0002441 A | 02-01-2001 |
| | | CN 1275376 A | 06-12-2000 |
| | | JP 2001011005 A | 16-01-2001 |
| | | US 2003162836 A1 | 28-08-2003 |
| EP 33614 A | 12-08-1981 | AR 228586 A1 | 30-03-1983 |
| | | AT 7782 T | 15-06-1984 |
| | | AU 6668771 A | 06-08-1981 |
| | | AU 537560 B2 | 28-06-1984 |
| | | AU 6668781 A | 06-08-1981 |
| | | CA 1195691 A1 | 22-10-1985 |
| | | DE 3162626 D1 | 12-07-1984 |
| | | DK 34781 A | 29-07-1981 |
| | | EP 0033614 A1 | 12-08-1981 |
| | | ES 508805 D0 | 16-11-1982 |
| | | ES 8300734 A1 | 01-02-1983 |
| | | ES 8300735 A1 | 01-02-1983 |
| | | GR 73505 A1 | 06-03-1984 |
| | | IE 810116 L | 28-07-1981 |
| | | JP 1040834 B | 31-08-1989 |
| | | JP 1556166 C | 23-04-1990 |
| | | JP 56150040 A | 20-11-1981 |
| | | US 4367238 A | 04-01-1983 |
| | | KR 8400239 B1 | 07-03-1984 |
| US 3600437 A | 17-08-1971 | AR 197075 A1 | 15-03-1974 |
| | | BE 737417 A | 13-02-1970 |
| | | CH 527155 A | 31-08-1972 |
| | | CH 537354 A | 31-05-1973 |
| | | CY 743 A | 30-04-1974 |
| | | DE 1941625 A1 | 19-11-1970 |
| | | DK 240477 A | 01-06-1977 |
| | | DK 240577 A ,B, | 01-06-1977 |
| | | DK 240677 A ,B, | 01-06-1977 |
| | | DK 145778 B | 28-02-1983 |
| | | ES 370540 A1 | 01-07-1971 |
| | | ES 390805 A1 | 16-03-1974 |
| | | FI 54099 B | 30-06-1978 |
| | | FI 762250 A ,B, | 05-08-1976 |
| | | FI 780846 A ,B, | 17-03-1978 |
| | | FR 2015728 A5 | 30-04-1970 |
| | | GB 1264340 A | 23-02-1972 |
| | | IE 33573 B1 | 21-08-1974 |
| | | IL 32825 A | 30-03-1973 |
| | | JP 51045586 B | 04-12-1976 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 03 40 5347

This annex lists the patent family membersrelating to the patent documents cited in the above-mentioned European search report.
The members are as contained in the European Patent Office EDP file on
The European Patent Office is in no way liable for these particulars which are merely given for the purpose of information.

06-10-2003

| Patent document cited in search report | Publication date | Patent family member(s) | Publication date |
|---|---|---|---|
| US 3600437 A | | JP 51044938 B | 01-12-1976 |
| | | LU 59302 A1 | 18-05-1970 |
| | | MY 13274 A | 31-12-1974 |
| | | NL 6912504 A ,B | 17-02-1970 |
| | | NL 7506127 A ,B, | 29-08-1975 |
| | | NL 7506128 A ,B, | 29-08-1975 |
| | | NL 7905644 A | 30-11-1979 |
| | | NO 132689 B | 08-09-1975 |
| | | NO 132689 C | 17-12-1975 |
| | | NO 137824 B | 23-01-1978 |
| | | NO 773495 A ,B, | 16-02-1970 |
| | | PH 9579 A | 19-01-1976 |
| | | PH 9478 A | 23-12-1975 |
| | | SE 398639 B | 09-01-1978 |
| | | SE 363818 B | 04-02-1974 |
| | | US 3972934 A | 03-08-1976 |
| | | US 4001322 A | 04-01-1977 |
| | | US 4062895 A | 13-12-1977 |
| US 5185377 A | 09-02-1993 | AT 156353 T | 15-08-1997 |
| | | AU 645599 B2 | 20-01-1994 |
| | | AU 8364091 A | 12-03-1992 |
| | | CA 2050569 A1 | 06-03-1992 |
| | | CS 9102718 A3 | 18-03-1992 |
| | | DE 59108814 D1 | 11-09-1997 |
| | | DK 474597 T3 | 20-10-1997 |
| | | EP 0474597 A2 | 11-03-1992 |
| | | ES 2106770 T3 | 16-11-1997 |
| | | FI 914185 A | 06-03-1992 |
| | | GR 3024440 T3 | 28-11-1997 |
| | | HU 58998 A2 | 28-04-1992 |
| | | HU 9500310 A3 | 30-10-1995 |
| | | IE 913110 A1 | 11-03-1992 |
| | | IL 99376 A | 19-01-1996 |
| | | JP 3264950 B2 | 11-03-2002 |
| | | JP 4257517 A | 11-09-1992 |
| | | KR 201749 B1 | 15-06-1999 |
| | | MX 9100917 A1 | 04-05-1992 |
| | | NO 913472 A | 06-03-1992 |
| | | PT 98874 A ,B | 31-07-1992 |
| | | SK 279104 B6 | 03-06-1998 |
| | | RU 2060731 C1 | 27-05-1996 |
| | | ZA 9107005 A | 29-04-1992 |
| WO 0250008 A | 27-06-2002 | AU 2395802 A | 01-07-2002 |
| | | WO 0250008 A2 | 27-06-2002 |

EPO FORM P0459

For more details about this annex : see Official Journal of the European Patent Office, No. 12/82

**ANNEX TO THE EUROPEAN SEARCH REPORT ON EUROPEAN PATENT APPLICATION NO.** EP 03 40 5347

This annex lists the patent family members relating to the patent documents cited in the above-mentioned European search report.
The members are as contained in the European Patent Office EDP file on
The European Patent Office is in no way liable for these particulars which are merely given for the purpose of information.

06-10-2003

| Patent document cited in search report | | Publication date | Patent family member(s) | Publication date |
|---|---|---|---|---|
| WO 0250008 | A | | US 2002128323 A1 | 12-09-2002 |

EPO FORM P0459

For more details about this annex : see Official Journal of the European Patent Office, No. 12/82